# EUROPEAN PATENT SPECIFICATION

(11) **EP 3 458 021 B1**
(45) Date of publication and mention of the grant of the patent: **09.07.2025**
(21) Application number: 17724858.0
(22) Date of filing: 12.05.2017
(51) Int. Cl.: A61K 8/37, A61Q 17/02, A61K 8/04, A61K 8/73, A61K 8/81, A61P 33/14

(54) **A LOUSE OVA AND NIT LOOSENING COMPOSITION**
LAUSEIER- UND -NISSENABLÖSUNGSZUSAMMENSETZUNG
COMPOSITIONS DE DÉCROCHAGE DES OEUFS DE POUX ET DES LENTES

(30) Priority: 17.05.2016 GB 201608647
(43) Date of publication of application: 27.03.2019
(73) Proprietor: Thornton & Ross Limited, Huddersfield HD7 5QH (GB)
(72) Inventor: COOPER, Nigel, Linthwaite Huddersfield HD7 5QH (GB); BRUNTON, Elizabeth, Rachel, Stow-Cum-Quy Cambridge CB25 9AU (GB)
(74) Representative: Meissner Bolte UK
(86) International application number: PCT/GB2017/000071
(87) International publication number: WO 2017/198982

(56) References cited:
- WO-A1-2013/030768
- WO-A1-2016/030851
- WO-A2-2009/040847
- FR-A1- 2 874 503
- US-A- 4 147 800
- US-A1- 2003 055 104
- US-A1- 2009 285 869

## Description

The present invention relates to a louse ova and nit loosening composition to enable louse ova and nits to be dislodged from the hair and combed off.

Many effective pediculicidal compositions are known for the treatment of head louse infestations. Some so-called "natural" or non-toxic remedies include sodium chloride or other alkali-metal halide salts, for example as described in US6601116. However, these tend to make the hair dry and are therefore unpleasant to use. While these and other toxic pediculicidal compositions that contain conventional insecticides, for example permethrin, may be effective against the adult parasites and have a significant mortality rate with regard to louse ova they do not assist in the removal of the louse ova and hatched egg cases, commonly called nits, from the hair. These lice ova and nits cling tenaciously to individual hairs, usually within 10 mm of the scalp, because they are effectively glued to the hair shaft by a hydrophobic glue that the louse secretes on to the hair shaft. The louse spreads the glue over the hair shaft by movement of her body and forms a mould into which she then lays the ovum, which is also substantially coated in the glue. The glue solidifies on exposure to oxygen in the atmosphere thereby firmly binding the ovum to the hair shaft.

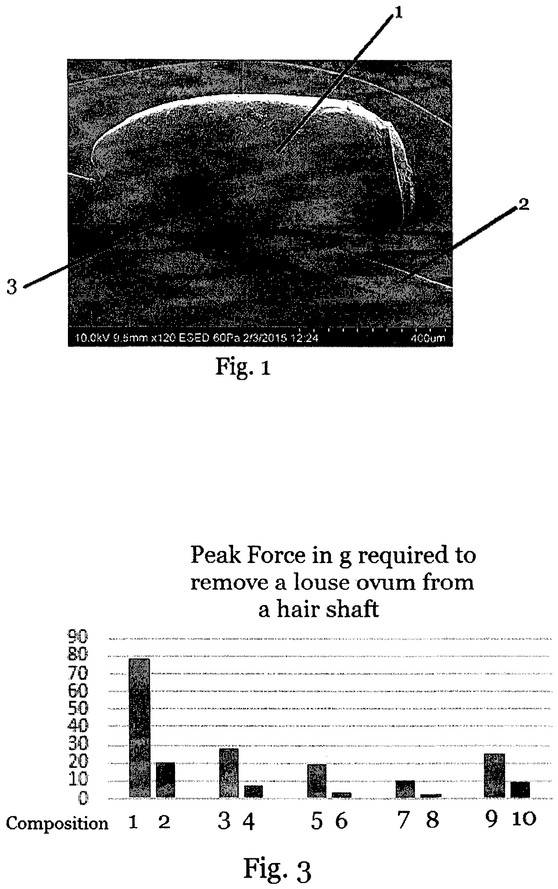

Fig. 1 is a photograph of an image produced by a scanning electron microscope of a louse ovum 1 attached to a human hair shaft 2. It can be seen that the ovum 1 is secured to the shaft 2 by a ligature 3 that surrounds the shaft 2 and is formed from the louse glue. The efficiency of this ligature is such that removal of the ovum 1 and resulting nit or egg-case from the hair shaft 2 after ovum 1 has hatched is extremely difficult.

Techniques conventionally used to remove lice ova and nits from the hair after treatment with pediculicidal compositions are the vigorous brushing of the hair or combing with a fine tooth nit comb. These techniques are inefficient and rarely succeed in removing a substantial quantity of the ova and nits because the louse glue is so effective. When used on their own, these techniques are painful and much more likely to damage the hair than remove the nits. They are also likely to cause stress and traumatize children being treated. Hence, hair conditioning oils are often applied to the scalp to lubricate the hair shafts prior to combing or brushing. Vinegar and other household acids have also been used either alone or in combination with hair conditioning oils with a view to softening the louse glue. However, none have been found to be substantially effective owing to the efficiency of the louse glue, which it is difficult to soften or destroy.

Other proprietary preparations have been proposed based on enzymes or solvents with a view to degrading the louse glue. For example, US 2003/055104 A1 discloses a nit removal composition in the form of a gel comprising 1-50% dimethylisosorbide in an aqueous vehicle with a thickening agent. WO 2013/030768 A1 discloses a composition for treating pediculosis comprising between 1 and 20% isopropyl myristate, between 1 and 20% silicone oil and at least 20% water.

US 4147800 A1 discloses a pediculicidal composition comprising an aliphatic alcohol and an aliphatic carboxylic ester wherein the ratio of alcohol to ester is between 1.1:1 and 3:1. However, as such preparations are primarily for use on children, it is not desirable that toxic or allergy-inducing chemicals be used. Also, there is no evidence that such preparations are effective.

WO 2016/030851 A1 to a composition, especially a cosmetic composition, comprising: at least one aqueous phase gelled by at least one hydrophilic gelling agent; and at least one oily phase gelled by at least one lipophilic gelling agent; said phases forming a macroscopically homogeneous mixture; said composition also comprising at least one anti-perspirant active agent selected from aluminium and/or zirconium salts, aluminium and/or zirconium complexes, and the mixtures thereof..

US 2009/285869 A2 describes a lecithin organogel ("LO") operates as a transdermal pharmaceutical delivery composition. In particular, the lecithin organogel comprises an internal oil phase containing oil-in-water ("O/W") and water-in-oil ("W/O") emulsifiers, and an aqueous phase comprising inorganic and organic hydrocolloids. The lecithin organogel may contain up to 80% additive ingredients, including biocompatible surfactants, nonpolar solvents, saturated fatty alcohols, moisturizers, preservatives or antimicrobials, and chelating agents.

FR 2874503 A1 describes a process for obtaining a firming active principle capable of combating stretch marks, derived from a solubilization of Cucurbita pepo seed cake treated by enzymatic hydrolysis. The invention also covers the active principle obtained as well as its uses in cosmetics.

WO 2009/040847 A2 describes a cosmetic compositions containing a combina¬ tion of functional ingredients of vegetal origin, one consisting of tamarind gum polysaccharide and the other consisting of a beech tree buds extract, which contains a pool of medium-low molecular weight molecules, among which flavonoids, polyphenols, water-soluble peptide fractions, amino-acids and mineral salts. Specifically, the compositions comprise from 0.1 % to 2% by weight of tamarind seed polysaccharide (Tamarindus lndica Seed Polysaccharide) and from 0.5 to 15% by weight of beech tree buds extract (Water (and) Fagus sylvatica Extract).

The object of the present invention is to provide a nit loosening composition that overcomes the deficiencies of conventional compositions and which is effective in loosening lice ova and nits from the hair.

An additional object of the present invention is to provide a nit removal composition that is safe for use on children, easy to use and relatively inexpensive.

Surprisingly, the applicant has found that conventional, non-toxic cosmetically- acceptable hair oils may be used in a composition, as indicated below, to produce results that are significantly better than when these oils are used neat. This also means that sodium chloride or other alkali-metal halide salts , enzymes and pharmacologically-active chemicals, which it may not be desirable to use on the hair, particularly that of a young child or infant, do not need to be used.

With regard to the above and the following description it should be appreciated the term "cosmetically-acceptable oil" covers such oils that on their own are essentially water insoluble and therefore immiscible with water.

Thus, according to the present invention there is provided a composition comprising a non - toxic sodium chloride-free aqueous gel containing at least 20% by weight of a cosmetically-acceptable oil being any or a mixture of isopropyl myristate, isopropyl palmitate and isononyl isononanoate, a thickener and at least 60% by weight water, wherein the cosmetically-acceptable oil is isononyl isononanoate, for use in the treatment of a human or an animal to loosen louse ova and nits.

It has been found that such an aqueous gel improves the performance of most hair conditioning oils as regards louse ova and nit removal from the hair over their performance when used neat. It has also been unexpectedly found that it has an ectoparasiticidal effect in addition, which is an advantage enabling it to be used both to kill lice and remove them and their nits from the hair.

Tests have been conducted using varying quantities of different cosmetically-acceptable oils in the composition. It was found that compositions containing 5% or less by weight of these oils were ineffective but that compositions containing in excess of 5% by weight and preferably at least 10% by weight of these oils were effective. Given that the oil has to be diluted in water and a thickener has to be present, quantities of these oils up to around 80% by weight are possible. However, the tests revealed that on average Optimum results were obtained when the cosmetically-acceptable oil was present in a quantity of 20% by weight and sufficiently diluted in water.

Advantageously, the composition has a pH in the range of 6 to 8 and is preferably pH neutral. This is a significant advantage over the use of household acids given that the composition is primarily going to be applied to children's heads and hair.

Whilst the invention has been found to produce a composition that improves the louse ova and nit loosening performance of the hair conditioning oil that it contains, it was also found that an oil consisting of any or a mixture of carboxylic acids or carboxylic esters gave some of the better results. Specifically, oils comprising any or a mixture of isopropyl myristate, isopropyl palmitate and isononyl isononanoate gave these better results and out of these isononyl isononanoate performed the best.

The thickener used may be a conventional cosmetically-acceptable thickener and is preferably selected from the group consisting of
(a) sodium acrylate/sodium acryloyldimethyl taurate copolymer
(b) xanthan gum, and
(c) polyacrylic acid.

Persons skilled in the art will recognize all of these thickeners as proprietary thickeners which are sold under the trade marks Creagel^{®} by Cosmetics Innovations and Technologies Sarl in the case of (a) and Carbopol^{®} by Lubrizol Advanced Materials, Inc. in the case of (c) above.

Preferably also, the thickener is an auto-emulsifier. Some thickeners thicken oils to produce gel emulsions. However, the advantage of an auto-emulsifier is that they are able to emulsify oil and water mixtures containing a large quantity of water. Such a thickener is sold under the trade mark Creagel^{®} EZ IN by Cosmetics Innovations and Technologies Sarl and comprises a gel comprised of a sodium acrylate/sodium acryloyldimethyl taurate copolymer and isononyl isononanoate.

An example of a preferred formulation of a louse ova and nit loosening composition comprising an auto-emulsifying thickener and in accordance with the invention comprises an aqueous gel wherein the thickener comprises a gel comprised of a sodium acrylate/sodium acryloyldimethyl taurate copolymer and isononyl isononanoate and the aqueous gel contains in addition 20% by weight of isononyl isononanoate as the cosmetically-acceptable oil and at least 60% water, isononyl isononanoate alone when used as the cosmetically-acceptable oil giving the best results as shown below.

In particular, a preferred specific example of a composition in accordance with the present invention is as follows.
20% by weight isononyl isononanoate
10% by weight Creagel^{®} EZ IN
Water to 100%

In some formulations the water content may be reduced by up to 5% to permit the addition of small quantities of colouring agents, fragrance agents and other cosmetically-acceptable additives. However, the formulation should not contain any pharmacologically-active ingredients. The composition is also pH neutral.

The compositions of the present invention may be prepared by any suitable means for producing hair gels. By way of example, the composition may be prepared by mixing the cosmetically-acceptable oil with any additives such as colouring and/or fragrance agents, diluting the resulting mixture with water and then thickening the diluted mixture with the thickener to produce the aqueous gel.

In use, the aqueous gel is applied to the hair and massaged in so that all hair shafts are coated. The gel should be left in place for a few minutes so that it commences work to loosen louse ova and nits. As it has been found that the gel also has an ectoparasiticidal effect most if not all live lice in the hair should be killed. The hair should then be combed with a fine-toothed nit comb to remove lice, loosened ova and nits, taking care to comb from hair roots to hair tips to ensure all ova and nits are acted on and satisfactorily removed from the hair shafts. Once the user is satisfied that all of the hair has been adequately combed, the gel can be washed out using a proprietary shampoo.

To justify the present invention the following tests were conducted to compare the force required to dislodge louse ova and nits from hair shafts after the application of various cosmetically-acceptable oils and various formulations in accordance with the present invention.

Fig. 2 is a diagram showing the set-up used in the test; and

Fig. 3 is a bar chart displaying the test results.

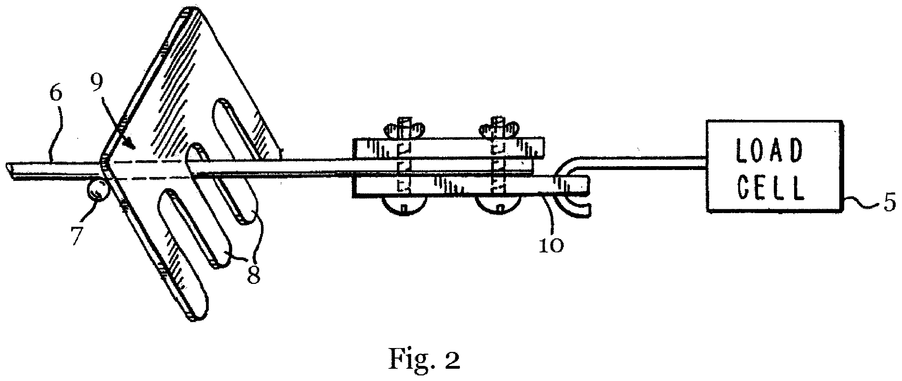

As shown in Fig. 2, the equipment used in the tests comprises a load cell 5 that was used to pull a hair shaft 6 to which a louse ovum 7 was attached between two teeth 8 of a fine tooth nit comb 9 via a clamp 10. The peak force required to dislodge the louse ovum 7 from the hair shaft 6 was measured after the hair shaft 6 and ovum 7 had been coated in one of the compositions used being tested. To take into account variations, identical tests were conducted at least 5 times using the same composition but different hairs and the average peak force was calculated.

The compounds used in the tests are indicated below and comprise neat oils, as listed; compositions including those in accordance with the present invention comprising 20% by weight of the oil in question, 10% by weight Creagelo EZ IN and water to 100%, listed as "composition using X" where X is the oil named; neat Creagelo EZ IN; and an emulsion of 20% by weight Creagelo EZ IN in water.
1 Creagel^{®} EZ IN (neat)
2. An emulsion of 20% by weight Creagel^{®} EZ IN with water to 100%
3. Isopropyl myristate
4. The composition using isopropyl myristate as the oil
5. Isopropyl palmitate
6. The composition using isopropyl palmitate as the oil
7. Isononyl isononanoate
8. The composition using isononyl isononanoate as the oil
9. A proprietary head louse treatment oil comprising a blend of essential oils and other plant extracts, namely oils of Vitis vinisera, Triticum vulgare, Azadirachta indica, Eucalyptus radiata, Pelargonium graveolens, Lavendula angustifolium, Rosmarinus officinalis and Melaleuca alternifolia.
10. The composition using the proprietary head louse treatment oil detailed at point 9. above as the oil

The average force required to dislodge the louse ovum from the hair shaft using the test method described above with the aforementioned compounds was as shown in the following table.

| **Compound** | **Peak force in g** |
|---|---|
| 1 | 78.45 |
| 2 | 20.25 |
| 3 | 28.175 |
| 4 | 7.6 |
| 5 | 19.45 |
| 6 | 4.0 |
| 7 | 10.37 |
| 8 | 2.275 |
| 9 | 25.27 |
| 10 | 9.65 |

The results of the tests detailed above are also shown in Fig. 3 in the form of a bar chart.

The test results show that compositions in accordance with the invention provided the best results. Also, the results for the thickener Creagelo EZ IN when alone and when in an emulsion with water show that the presence of the cosmetically-acceptable oil is required to produce the significant improvement in the louse ova and nit loosening capability found.

It will be seen that the best results were obtained using isopropyl palmitate as the cosmetically-acceptable oil.

Further research will be necessary to explain the significant increase in performance of compositions in accordance with the present invention over the use of neat oils as it is not known with certainty how these compositions work. It is thought that dilution of the oil in a thickened gel enables the oil to coat and to penetrate the louse glue more effectively. This penetration degrades the glue and thereby slackens the ligature retaining the louse ovum or nit to the hair shaft. This enables the louse ovum or nit to slide off the hair shaft when combed, the composition also providing lubrication for this purpose.

## Claims

1. A composition comprising a non-toxic sodium chloride-free aqueous gel containing at least 20% by weight of a cosmetically-acceptable oil being any or a mixture of isopropyl myristate, isopropyl palmitate and isononyl isononanoate, a thickener and at least 60% by weight water, wherein the cosmetically-acceptable oil is isononyl isononanoate, for use in the treatment of a human or an animal to loosen louse ova and nits.

2. A composition for use as claimed in Claims 1, wherein the composition has a pH of between 6 and 8.

3. A composition for use as claimed in Claim 1 or Claim 2, wherein the composition is pH neutral.

4. A composition for use as claimed in any of Claims 1 to 3, wherein the thickener is selected from the group consisting of
(a) sodium acrylate/sodium acryloyldimethyl taurate copolymer and isononyl isononanoate,
(b) xanthan gum, and
(c) polyacrylic acid.

5. A composition for use as claimed in any of Claims 1 to 4, wherein the thickener is an auto-emulsifier.

6. A composition for use as claimed in Claim 5, wherein the thickener comprises a gel comprised of a sodium acrylate/sodium acryloyldimethyl taurate copolymer and isononyl isononanoate.

7. A composition for use as claimed in any of Claims 1 to 6, wherein the thickener comprises a gel comprised of a sodium acrylate/sodium acryloyldimethyl taurate copolymer and isononyl isononanoate, and the aqueous gel contains in addition 20% by weight of either or a mixture of isononyl isononanoate and isopropyl palmitate as the cosmetically-acceptable oil and at least 60% by weight water.

8. A composition for use as claimed in any of Claims 1 to 7, comprised of
20% by weight isononyl isononanoate;
10% by weight of a gel comprised of sodium acrylate/sodium acryloyldimethyl taurate copolymer and isononyl isononanoate;
between 0% and 5% cosmetically-acceptable additive; and
water to 100%.

## Patentansprüche

1. Zusammensetzung, umfassend ein ungiftiges natriumchloridfreies wässriges Gel, das mindestens 20 Gew.% eines kosmetisch annehmbaren Öls, das beliebige von oder eine Mischung von Isopropylmyristat, Isopropylpalmitat und Isononylisononanoat ist, ein Verdickungsmittel und mindestens 60 Gew.% Wasser enthält, wobei das kosmetisch annehmbare Öl Isononylisononanoat ist, zur Verwendung in der Behandlung eines Menschen oder eines Tieres, um Lauseier und -nissen abzulösen.

2. Zusammensetzung zur Verwendung nach Anspruch 1, wobei die Zusammensetzung einen pH-Wert zwischen 6 und 8 hat.

3. Zusammensetzung zur Verwendung nach Anspruch 1 oder Anspruch 2, wobei die Zusammensetzung pH-neutral ist.

4. Zusammensetzung zur Verwendung nach einem der Ansprüche 1 bis 3, wobei das Verdickungsmittel ausgewählt ist aus der Gruppe bestehend aus
(a) Natriumacrylat/Natriumacryloyldimethyltaurat-Copolymer und Isononylisononanoat,
(b) Xanthangummi und
(c) Polyacrylsäure.

5. Zusammensetzung zur Verwendung nach einem der Ansprüche 1 bis 4, wobei das Verdickungsmittel ein Auto-Emulgator ist.

6. Zusammensetzung zur Verwendung nach Anspruch 5, wobei das Verdickungsmittel ein Gel umfasst, das aus einem Natriumacrylat/Natriumacryloyldimethyltaurat-Copolymer und Isononylisononanoat zusammengesetzt ist.

7. Zusammensetzung zur Verwendung nach einem der Ansprüche 1 bis 6, wobei das Verdickungsmittel ein Gel umfasst, das aus einem Natriumacrylat/Natriumacryloyldimethyltaurat-Copolymer und Isononylisononanoat zusammengesetzt ist, und das wässrige Gel zusätzlich 20 Gew.% von einem von oder einer Mischung von Isononylisononanoat und Isopropylpalmitat als das kosmetisch annehmbare Öl und mindestens 60 Gew.% Wasser enthält.

8. Zusammensetzung zur Verwendung nach einem der Ansprüche 1 bis 7, die zusammengesetzt ist aus
20 Gew.% Isononylisononanoat;
10 Gew.% eines Gels, das aus Natriumacrylat/Natriumacryloyldimethyltaurat-Copolymer und Isononylisononanoat zusammengesetzt ist,
zwischen 0 % und 5 % kosmetisch annehmbarem Additiv; und
Wasser auf 100 %.

## Revendications

1. Composition comprenant un gel aqueux sans chlorure de sodium non toxique contenant au moins 20 % en poids d'une huile cosmétiquement acceptable qui est l'un quelconque parmi le myristate d'isopropyle, le palmitate d'isopropyle et l'isononanoate d'isononyle ou un mélange de ceux-ci, un épaississant et au moins 60 % en poids d'eau, dans laquelle l'huile cosmétiquement acceptable est l'isononanoate d'isononyle, pour une utilisation dans le traitement d'un être humain ou d'un animal pour décrocher des œufs de poux et des lentes.

2. Composition pour une utilisation selon la revendication 1, dans laquelle la composition a un pH compris entre 6 et 8.

3. Composition pour une utilisation selon la revendication 1 ou la revendication 2, dans laquelle la composition est de pH neutre.

4. Composition pour une utilisation selon l'une quelconque des revendications 1 à 3, dans laquelle l'épaississant est choisi dans le groupe constitué par
(a) un copolymère d'acrylate de sodium/taurate d'acryloyldiméthyle sodique et d'isononanoate d'isononyle,
(b) la gomme de xanthane, et
(c) le polyacide acrylique.

5. Composition pour une utilisation selon l'une quelconque des revendications 1 à 4, dans laquelle l'épaississant est un auto-émulsifiant.

6. Composition pour une utilisation selon la revendication 5, dans laquelle l'épaississant comprend un gel composé d'un copolymère d'acrylate de sodium/taurate d'acryloyldiméthyle sodique et d'isononanoate d'isononyle.

7. Composition pour une utilisation selon l'une quelconque des revendications 1 à 6, dans laquelle l'épaississant comprend un gel composé d'un copolymère d'acrylate de sodium/taurate d'acryloyldiméthyle sodique et d'isononanoate d'isononyle, et le gel aqueux contient en outre 20 % en poids de l'un ou l'autre ou d'un mélange d'isononanoate d'isononyle et de palmitate d'isopropyle en tant qu'huile cosmétiquement acceptable et au moins 60 % en poids d'eau.

8. Composition pour une utilisation selon l'une quelconque des revendications 1 à 7, composée de
20 % en poids d'isononanoate d'isononyle ;
10 % en poids d'un gel composé d'un copolymère d'acrylate de sodium/taurate d'acryloyldiméthyle sodique et d'isononanoate d'isononyle ;
entre 0 % et 5 % d'un additif cosmétiquement acceptable ; et
de l'eau jusqu'à 100 %.
